# EUROPEAN PATENT APPLICATION

(11) **EP 0 594 253 A1**
(43) Date of publication of application: **27.04.1994**
(21) Application number: 93202908.5
(22) Date of filing: 19.10.1993
(51) Int. Cl.: A61F 5/455, A61F 5/44

(54) **Feminine urinary device, allowing women to urinate in an upright posture**

(30) Priority: 22.10.1992 IT MI922412
(71) Applicant: SANIPLAST s.r.l., I-20123 Milano (IT)
(72) Inventor: Merry del Val, Domingo, I-22030 Montorfano (Como) (IT)
(74) Representative: Faraggiana, Vittorio, Dr. Ing.

(57) **Abstract**

A device allowing women to urinate in an upright posture consisting in a duct element (10), of substantially conical shape, with an oblique larger base (11), made of a flexible material. Furthermore, appropriate clothes are foreseen to facilitate the use of the device such as, for instance, trousers with a front opening extended to the middle of the crotch for the introduction of the device itself. Similarly, underwear with appropriate openings or flaps can be foreseen.

## Description

The present invention refers to a device suitable for allowing a woman to urinate in un upright posture.

For hygienical reasons, this purpose is frequently desired and needed, specially when public toilets are used.

The device is also greatly useful to women, particularly when carrying out jobs or playing sports which require special, typically male clothes (trousers, overalls, tracksuits, skisuits, etc.).

In view of said object, it has been thought out, according to the invention, a device allowing women to urinate in an upright posture, consisting of a duct element, of substantially conical shape, with an oblique larger base, made of a flexible material.

Furthermore, a garment to be used with the above mentioned device has been also studied. The front opening of this garment is extended at least to the middle of the crotch, to allow the introduction of the device.

To better explain the innovative principles of this invention and its advantages with respect to the prior art, an exemplifying embodiment - applying these principles - is described hereunder, with the aid of the accompanying drawings. In the drawings:
- figure 1 shows a schematic perspective-view of the duct element according to the invention;
- figure 2 shows a schematic view of the duct element shown in figure 1, in the position of use;
- figure 3 shows a schematic perspective-view of a garment suitable for facilitating the use of the duct element shown in figure 1;
- figure 4 shows a partial view of a panty-hose modified so as to be used with the duct element shown in figure 1.

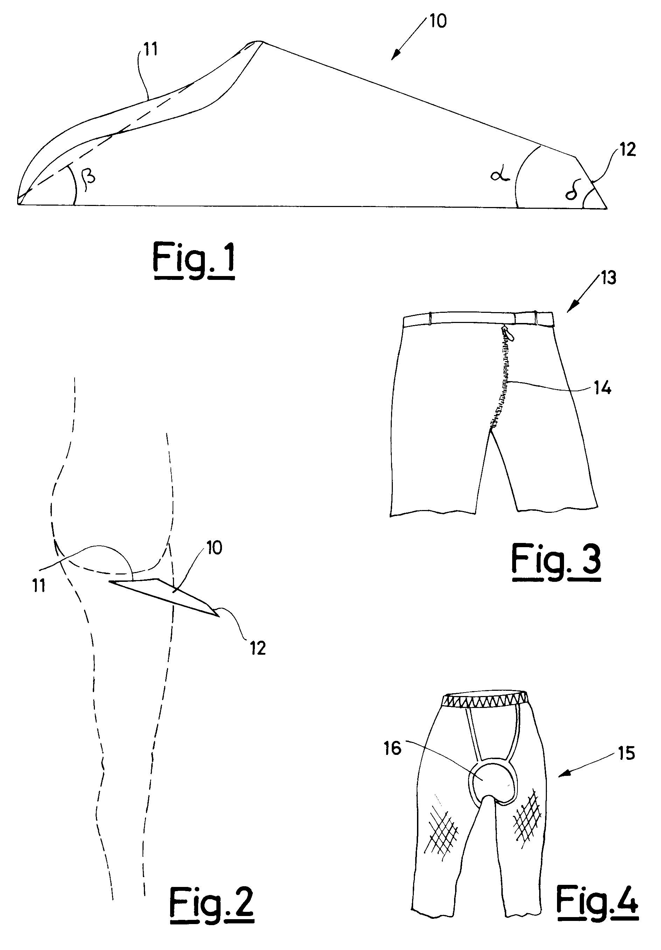

With reference to the drawings, figure 1 shows a duct element or device according to the invention, generically indicated with 10. This duct element has a generically conical shape, and comprises a wide inlet end, or larger base 11, slanting with respect to the axis of the cone, and an outlet end or smaller base 12, with a slanting opening, opposite to the larger base to provide a pouring lip.

The duct element can be advantageously made of flexible material, such as waterproof paper, adequately stiffened, normally flattened on the two opposite generatrixes of the cone (generally the upper one and the lower one, as shown in figure 1), to be opened in a tubular manner at the time of use.

The use of paper or thin cardboard as manufacturing material is economically profitable, so as to allow the manufacturing of disposable devices.

Approximately, the following sizes turned out to be advantageous: lenght of the longer generatrix of the cone between 130mm and 230mm, preferably around 180mm; angle α of opening of the cone between 12° and 40°, preferably around 20°; inclination angle β of the larger base with respect to the longer generatrix of the cone between 15° and 50°, preferably around 35°; angle δ of the smaller base or outlet lip with respect to the longer generatrix between 45° and 80°, preferably around 55°.

Figure 2 schematically shows the duct element places between the legs so as to allow the inlet end 11 to collect the urine and convey it, due to the inclination of the cone downwards, towards the outlet end 12, thus allowing a woman to satisfy her physiological needs standing upright.

For a more comfortable use of the device 10, it is possible to carry out garments specially studied to facilitate the positioning of the duct element.

For instance, figure 1 schematically shows a garment, such as a pair of trousers 13, having a zip 14 longer than usual to reach at least the middle of the crotch, thus permitting a comfortable introduction of the device 10.

For example, the opening can be extended up to 3 cm after the seam joining the front and the back parts of the leg of the trousers.

The word "trousers" is here used in the widest sense of the term, meaning with this also the inferior part of overalls or similar such as, for instance, tracksuits, skisuits, divided skirts, shorts, etc.

The realization of a series of garments and underwear having an opening under the crotch so as to permit the introduction of the device is of course foreseeable, thus allowing women to use the device and not to be obliged - as it happens today - to completely undress the lower part of their body.

For example, figure 4 shows a panty-hose 15 with a front opening 16 for the introduction of the device.

It is also possible to carry out slips or panty-hose with flaps, or other adjustments, similar to the ones used for male underwear.

Obviously, the above description of an embodiment applying the innovative principles of this invention is hereby reported only for illustrating purpose, and therefore must not be considered as a limitation of the claimed invention.

For example, the real proportions of the duct element and the shape of the inlet and outlet ends can vary from the ones shown in the drawings.

## Claims

1. Device allowing women to urinate in an upright posture, consisting of a duct element (10) of substantially conical shape, with an oblique larger base (11), made of a flexible material.

2. Device according to claim 1, characterized in that the duct element (10) has a smaller base (12) obliquely opposite to the larger base (11).

3. Device according to claim 1, characterized in that the duct element (10) has length of the longer generatrix of the cone between 130mm and 230mm, preferably around 180mm.

4. Device according to claim 1, characterized in that the duct element (10) has angle α of opening of the cone between 12° and 40°, preferably around 20°.

5. Device according to claim 1, characterized in that the duct element (10) has angle β of inclination of the larger base with respect to the longer generatrix between 15° and 50°, preferably around 35°.

6. Device according to claim 1, characterized in that the duct element (10) has angle δ of inclination of the smaller base with respect to the longer generatrix between 45° and 80°, preferably around 55°.

7. Garment to be used with the device according to claim 1, with anterior opening prolonged at least up to the middle of the crotch, for the introduction of the device.

8. Garment according to claim 7, characterized in that said opening is closable with a zip.

9. Garment according to claim 7, characterized in that the garment is a pair of trousers with the front opening extended at least 3 cm after the seam which joins the front and back parts of the leg of the trousers.

10. Garment according to claim 7, characterized in that the garment is a slip or panty-hose with a flap carrying out the opening.
